# EUROPEAN PATENT APPLICATION

(11) **EP 3 219 309 A1**
(43) Date of publication of application: **20.09.2017**
(21) Application number: 16160794.0
(22) Date of filing: 17.03.2016
(51) Int. Cl.: A61K 9/20, A61K 31/4184, A61K 31/4422, A61K 31/549

(54) **FIXED DOSED PHARMACEUTICAL COMPOSITION COMPRISING AMLODIPINE, CANDESARTAN CILEXETIL AND HYDROCHLOROTHIAZIDE FOR THE TREATMENT OF HYPERTENSION**

(71) Applicant: K.H.S. Pharma Holding GmbH, 55218 Ingelheim am Rhein (DE)
(72) Inventor: Jünemann, Daniel, 65346 Eltville (DE); Kahm, Andreas, 55218 Ingelheim (DE)
(74) Representative: Drescher, Christian

(57) **Abstract**

The present invention relates to fixed dose pharmaceutical compositions comprising the active substances Candesartan cilexetil, Amlodipine and Hydrochlorothiazide for the treatment of hypertension.

## Description

### BACKGROUND OF THE INVENTION

The present invention relates to fixed dose pharmaceutical compositions comprising the three active pharmaceutical ingredients (APls) Amlodipine, Candesartan cilexetil and Hydrochloro-thiazide for the treatment of hypertension.

Candesartan is the international nonproprietary name (INN) of 2-ethoxy-1-({4-[2-(2*H*-1,2,3,4-tetrazol-5-yl)phenyl]phenyl}methyl)-1*H-*1,3-benzodiazole-7-carboxylic acid which has the following chemical structure:

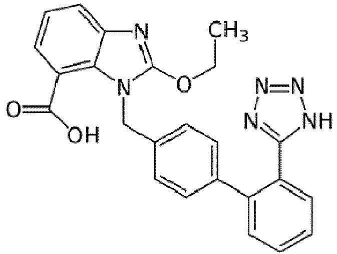

Hydrochlorothiazide (abbreviated "HCTZ" or "HCT") is the INN of 6-chloro-1,1-dioxo-3,4-dihydro-2*H*-1,2,4-benzothiadiazine-7-sulfonamide having the following chemical structure:

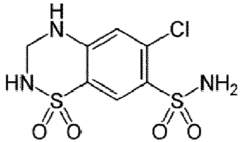

Amlodipine is the INN of (*RS*)-3-ethyl-5-methyl-2-[(2-aminoethoxy)methyl]-4-(2-chloro--phenyl)-6-methyl-1,4-dihydropyridine-3,5-dicarboxylate with the following chemical structure:

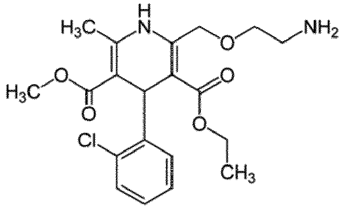

Pharmaceutical compositions of the angiotensin II antagonist Candesartan in the modification of its prodrug Candesartan cilexetil are registered for the treatment of congestive heart failure, diabetic nephropathies, diabetic retinopathy and hypertension, being marketed under the trade name ATACAND^{®}. Pharmaceutical compositions of the diuretic Hydrochlorothiazide are registered and marketed under the trade name ESIDRIX^{®} for the treatment of hypertension, edema and heart failure. Pharmaceutical compositions of the calcium channel blocker Amlodipine in the modification of its benzene sulfonate ("besilate") salt are registered and marketed under the trade name NORVASC^{®} for the treatment of coronary artery disease and hypertension.

A fixed-dose combination of Candesartan cilexetil and Hydrochlorothiazide is registered and marketed under the trade name ATACAND^{®} PLUS for the treatment of hypertension.

No fixed-dose pharmaceutical formulation comprising Candesartan cilexetil, Hydrochlorothiazide and Amlodipine has been registered and marketed yet.

**Table 1**

| **Brand name(s)** | NORVASC^{®} | ATACAND^{®} | ATACAND PLUS^{®} | ESIDRIX^{®} |
|---|---|---|---|---|
| **Originator company** | Pfizer | Astra Zeneca | | Novartis |
| **Active(s)** | Amlodipine besilate | Candesartan cilexetil | Candesartan cilexetil Hydrochlorothiazide | Hydrochlorothiazide |
| **Composition (qualitative)** | Microcrystalline cellulose, Calcium hydrogen phosphate anhydrous, Sodium starch glycolate Type A, Magnesium stearate | Carmellose-Calcium, Hyprolose, Lactose monohydrate, Magnesium stearate, Maize starch, Macrogol 8000, Ferric oxide | Carmellose-Calcium, Hyprolose, Lactose monohydrate, Magnesium stearate, Maize starch, Macrogol 8000, Ferric oxide | Lactose monohydrate, Magnesium stearate, Highly dispersed silicon dioxide, Wheat starch, Talc |
| **Strengths** | 5, 10 mg | 4, 8, 16, 32* mg | 8/12.5 mg, 16/12.5 mg, 32/12.5 mg, 32/25 mg | 12.5 mg, 25 mg |

| | | | | |
|---|---|---|---|---|
| * marketed as ATACAND PROTECT^{®} | | | | |

In the development of such triple-fixed-dose combinational products several problems have to be overcome:
Candesartan cilexetil is known for its low bioavailability and high tendency to undergo hydrolysis and dealkylation. Amlodipine besilate, on the other hand, is highly hygroscopic and sensitive to hydrolysis, transesterification of the ester groups and oxidation of the dihydropyrine ring. Candesartan cilexetil and Amlodipine besilate are susceptible to static electricity, whereas all three APIs exhibit poor flow properties.

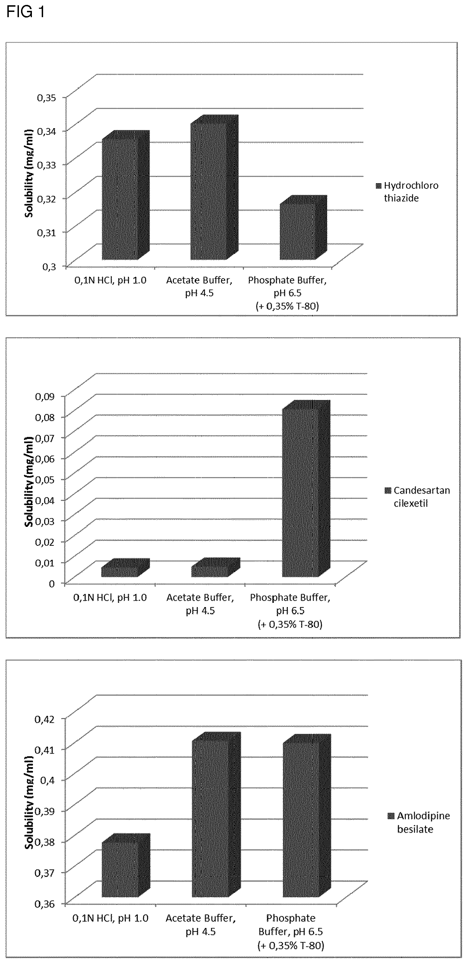

As visualized in figure 1, all three active substances exhibit quite different solubility profiles. Candesartan cilexetil, for example, is barely soluble at low pH-values, whereas the solubility of Hydrochlorothiazide decreases with increasing pH-values.

As assembled in table 1, the excipients and compositions of NORVASC^{®} (active: Amlodipine besilate) ESIDRIX^{®} (active: HCTZ) and ATACAND^{®} (active: Candesartan cilexetil) are quite different from each other.

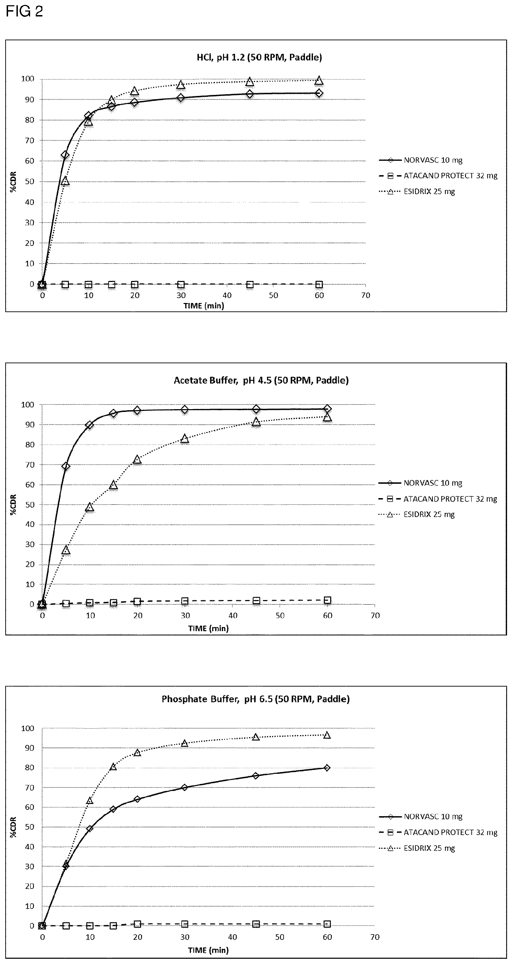

As can be seen in figure 2, the three different (mono) brand products also show different dissolution characteristics in standard media, i.e. in HCl at pH 1.2, in acetate buffer at pH 4.5, and phosphate buffer at pH 6.5. The solubility of Candesartan cilexetil is so low that a detergent has to be added to allow the media to show any discriminatory effect. The respective recommended standard media are displayed in table 6.

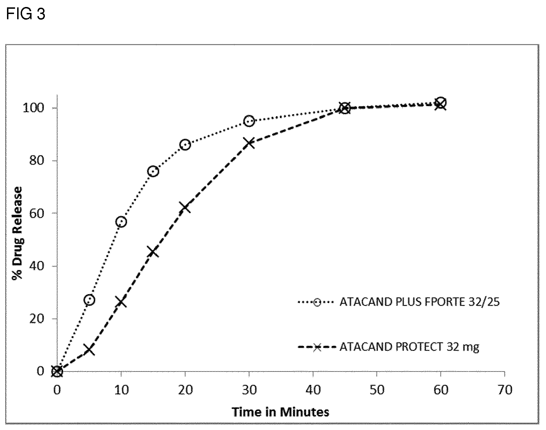

As can be deducted from figure 3, a comparison of the release rates of Candesartan cilexetil from ATACAND^{®} and Candesartan cilexetil from ATACAND PLUS^{®} - both having the same qualitative composition of excipients - reveals that the dissolution profiles are significantly different.

As can be seen in table 1 and table 2, sixteen (16) different fixed-dose combinations with different strengths of Amlodipine, Candesartan cilexetil and Hydrochlorothiazide are theoretically possible.

**Table 2**

| **Fixed dose combination** | **Amlodipine** | **Candesartan cilexitil** | **Hydrochlorothiazide** |
|---|---|---|---|
| 1. | 5 mg | 4 mg | 12.5 mg |
| 2. | 10 mg | | |
| 3. | 5 mg | 8 mg | |
| 4. | 10 mg | | |
| 5. | 5 mg | 16 mg | |
| 6. | 10 mg | | |
| 7. | 5 mg | 32 mg | |
| 8. | 10 mg | | |
| 9. | 5 mg | 4 mg | 25 mg |
| 10. | 10 mg | | |
| 11. | 5 mg | 8 mg | |
| 12. | 10 mg | | |
| 13. | 5 mg | 16 mg | |
| 14. | 10 mg | | |
| 15. | 5 mg | 32 mg | |
| 16. | 10 mg | | |

For several technical, regulatory and commercial reasons it is economically not attractive to develop, register and manufacture all 16 different fixed-dose combinations of Amlodipine, Candesartan cilexetil and Hydrochlorothiazide. It is thus necessary to focus on those fixed-dose combinations which allow for a cost efficient registration process as well as economical manufacturing.

In summary, it can be concluded that there is no guidance in the prior art how to design, develop and manufacture a fixed dose combination of Amlodipine, Candesartan cilexetil and Hydrochlorothiazide and to deal with all the obstacles described above.

It has now been found that fixed dose pharmaceutical formulations of Amlodipine, Candesartan cilexetil and Hydrochlorothiazide according to the present invention satisfy all requirements as regards the stability and the processability of all three active substances as well as their release and dissolution profile compared to their respective brand products.

### BRIEF DESCRIPTION OF THE DRAWINGS

**Figure 1** shows the solubility of Hydrochlorothiazide, Candesartan cilexetil and Amlodipine besilate in various standard dissolution media.
**Figure 2** shows the dissolution of ESIDRIX^{®} (Hydrochlorothiazide), ATACAND^{®} (Candesartan cilexetil) and NORVASC^{®} (Amlodipine besilate) in various standard dissolution media.
**Figure 3** shows a comparison of the dissolution profiles of ATACAND PROTECT^{®} (32 mg Candesartan mono) versus ATACAND PLUS FORTE^{®} (32 mg Candesartan + 25 mg Hydrochlorothiazide) under the respective recommended standard conditions.
**Figure 4a** shows a comparison of the dissolution profiles of Amlodipine besilate from formulation I, formulation II and NORVASC^{®}.
**Figure 4b** shows a comparison of the dissolution profiles of Candesartan cilexetil from formulation I, formulation II and ATACAND^{®}.
**Figure 5** shows a comparison of the dissolution profiles of Candesartan cilexetil having different particle size distributions from formulation I.
**Figure 6** shows a flow chart of the manufacturing process for formulations I, Ia, Ib and Ic.

### DETAILED DESCRIPTION OF THE INVENTION

The present invention relates to fixed dose pharmaceutical compositions comprising the three active pharmaceutical ingredients (APIs) Amlodipine, Candesartan cilexetil and Hydrochlorothiazide for the treatment of hypertension. In a preferred embodiment of the present invention, Amlodipine is utilized in the form of its benzenesulfonate (besilate) salt.

Amlodipine besilate may be manufactured according to processes known in the art, e.g. as disclosed in patent application EP 0 244 944 A2. Candesartan cilexetil may also be manufactured according to processes known in the art, e.g. as disclosed in patent application EP 0 459 136 A1. Hydrochlorothiazide may also be manufactured according to processes known in the art, e.g. as disclosed in patent US 3,163,645.

The pharmaceutical compositions according to the present invention, preferably in the form of tablets, comprise Candesartan cilexetil and a first matrix of excipients, Amlodipine and a second matrix of excipients, comprising at least one filler and Hydrochlorothiazide which may be comprised in either matrix.

Preferably, the first matrix is in the form of granules comprising Candesartan cilexetil, one or more disintegrants, one or more binders, one or more fillers, one or more stabilizers and optionally one or more glidants and other pharmaceutically acceptable excipients; whereas the second extragranular matrix comprises Amlodipine, one or more fillers, one or more lubricants and optionally other pharmaceutically acceptable excipients. Hydrochlorothiazide may be present either in the first granular matrix or in the second extragranular matrix.

In the context of the present invention, the terms actives, fillers, disintegrants, stabilizers, binders, lubricants, glidants etc. shall be understood as including a single compound but also multiple compounds.

Pharmaceutically acceptable disintegrants include, but are not limited to, carboxymethyl cellulose (carmellose), sodium starch glycolate, polyvinylpyrrolidone, crospovidone, croscarmellose, low substituted hydroxypropyl cellulose (L-HPC) and mixtures thereof. The preferred disintegrant is carmellose calcium.

In a preferred embodiment of the present invention, the one or more disintegrants are completely intragranular. It was found that the amount of intragranular disintegrant should be kept in a certain range. If the amount of disintegrant is below 1.0 wt.-%, Candesartan cilexetil is released too slowly. On the other hand, an amount of disintegrant of 5.0 wt.-% or higher results in a too fast dissolution of Candesartan cilexetil. Accordingly, the amount of disintegrants is preferably in the range from 1.5 wt.-% to 4.0 wt.-%, relative to the total weight of the composition.

Pharmaceutically acceptable binders include, but are not limited to, hydroxypropyl cellulose (HPC), hydroxypropyl methylcellulose (HPMC), dihydroxypropyl cellulose, methyl cellulose, hydroxyethyl cellulose, ethyl cellulose, sodium carboxymethyl cellulose, polyethylene glycol, maltodextrin, pregelatinized starch, polymethacrylates, sodium alginate, polyvinylpyrrolidone (povidone) and vinylpyrrolidone/vinylacetate copolymer (copovidone) and mixtures thereof. The preferred binder is hydroxypropyl cellulose (HPC).

It has been found that a certain minimum of binder is necessary for the formation of granules, whereas too high amounts lead to poor dissolution of the API(s). Accordingly, in a preferred embodiment of the invention, the amount of one or more binder(s) is in the range from 1 wt.-% to 10.0 wt.-%, more preferably in the range from 6.0 wt.-% to 9.0 wt.-%. In a particularly preferred embodiment of the invention, the binder is incorporated into the granular matrix only.

To match the different dissolution profiles of the three different active substances at the same time with a fixed dose combination/formulation is not trivial, as already indicated in figure 2.

It has been found that not only the total amount of disintegrants and binders does matter, but also their relative proportions.

For some reason, changes of the composition of the granular matrix appear to affect also the dissolution of Amlodipine being located in the extragranular matrix.

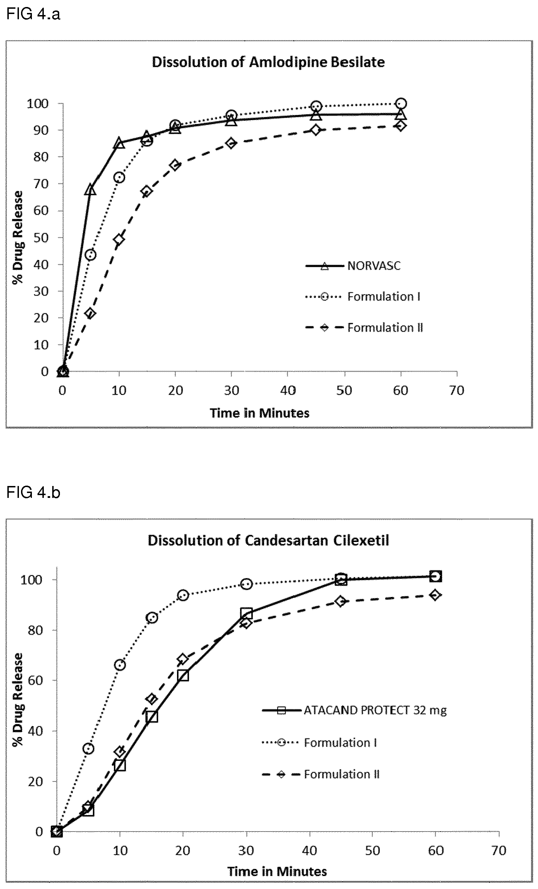

As demonstrated in figure 4a, the release of Amlodipine from formulation I, having a ratio of about 1.0:1.5 between disintegrant and binder, is slower compared to the reference product NORVASC^{®}. However, the release of Candesartan cilexetil from this formulation is faster compared to the reference product ATACAND^{®}, as demonstrated in figure 4b.

In order to slow down the release of Candesartan cilexetil, the amount of disintegrant was reduced whereas the amount of binder was increased in formulation II compared to formulation I, resulting in a ratio of about 1.0:4.0 between disintegrant and binder.

As demonstrated in figure 4b, the release of Candesartan cilexetil from formulation II is faster compared to formulation I. However, the release of Amlodipine from formulation II is slower compared to formulation I, as demonstrated in figure 4a. This finding is unexpected because the change of binder and disintegrant occurred in the granular matrix, whereas the composition of the extragranular matrix, comprising the Amlodipine, was not changed.

Interestingly, it appears that the dissolution profile of Hydrochlorothiazide is not influenced by changes of the composition. On the contrary, even the transfer of the full amount of Hydrochlorothiazide from the granular matrix into the extragranular matrix does not change its dissolution profile significantly.

As a result from the observations above, it can be said that both formulations are satisfactory as regards the approach of the dissolution profiles of all reference products NORVASC^{®}, ATACAND^{®} and ESIDRIX^{®} in order to achieve bioequivalence for all active substances. However, special attention has to be drawn towards the ratio between the amount of disintegrants relative to the amount of binders in the formulation, which should be kept in the range of from 1.0:1.5 to 1.0:4.0 by weight.

Accordingly, preferred embodiments of the present invention are characterized by a ratio between the amount of disintegrants and the amount of binders in the formulation that is in the range of from 1.0:1.5 to 1.0:4.0 by weight.

Pharmaceutically acceptable fillers include, but are not limited to, lactose, starch, microcrystalline cellulose (MCC), sucrose, mannitol, dicalcium phosphate, calcium carbonate, magnesium carbonate, low substituted hydroxypropyl cellulose (L-HPC), powder cellulose, calcium silicate, calcium phosphate, sorbitol, dextrin, kaolin, magnesium oxide, calcium sulfate, xylitol, isomalt, glucose, fructose, maltose and mixtures thereof. Preferred filler(s) are lactose monohydrate and maize starch, microcrystalline cellulose (MCC) and mixtures thereof.

The total amount of fillers is preferably in the range between 30 wt.-% and 85 wt.-%, based on the total weight of the composition.

In view of the finding that the composition of the granular matrix impacts also the characteristics of the extragranular matrix, it was quite surprising that even high amounts of lactose in the granular matrix appears not to have any impact on the stability of extragranular Amlodipine, which is known to readily form colored impurities when exhibited to lactose because of the Maillard reaction. Accordingly, a preferred embodiment of the present invention is characterized by a content of 50 wt.-% or more of lactose monohydrate.

Preferred pharmaceutical compositions according to the present invention comprise one or more stabilizers. The most preferred stabilizing agent for Candesartan cilexetil is polyethylene glycol (PEG). Since high amounts of PEG are known to result in increased impurities (i.e. Impurity D), the amount of PEG should be kept as low as possible. It has now been found that PEG in amounts (by weight) of as low as a ratio of 1.0:6.0 to 1.0:8.0 relative to Candesartan cilexetil does not only sufficiently stabilize Candesartan cilexetil, but furthermore leads to formulations with an even improved processability.

Accordingly, preferred pharmaceutical formulations according to the present invention comprise PEG in amounts (by weight) of as low as a ratio of 1.0:6.0 to 1.0:8.0 relative to Candesartan cilexetil.

Suitable lubricants include, but are not limited to, magnesium stearate, calcium stearate, sodium stearate, stearic acid, sodium glyceryl behenate, hexanedioic acid, hydrogenated vegetable oil sodium, stearyl fumarate, glycerin fumarate and mixtures thereof. The preferred lubricant is magnesium stearate. The amount of lubricants is preferably in the range between 0.5 wt.-% and 2.0 wt.-%, relative to the total weight of the composition, preferably being present in the extragranular matrix only.

The pharmaceutical compositions according to the present invention may optionally comprise pharmaceutically acceptable glidants such as colloidal silicon dioxide, talcum, magnesium carbonate and mixtures thereof. The amount of glidant(s) is preferably in the range between 0.1 wt.-% and 1.5 wt.-%, relative to the total weight of the composition.

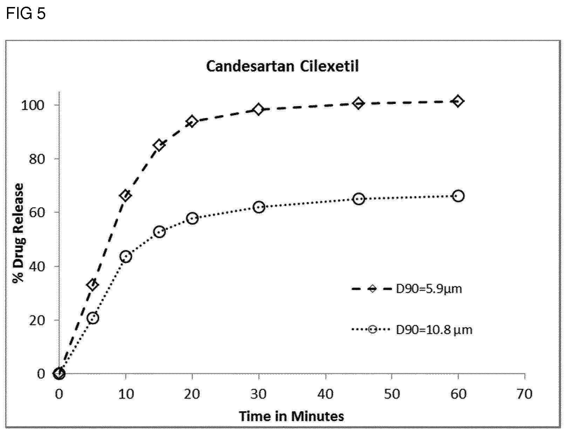

The dissolution profile of pharmaceutical formulations of Candesartan cilexetil is somewhat affected by the size of the Candesartan cilexetil particles. As indicated in figure 5, formulation I equipped with Candesartan cilexetil having a particle size distribution with a D₉₀-value of about 10.8 µm shows an incomplete total drug release, whereas the drug release from formulation I equipped with Candesartan cilexetil particles having a particle size distribution with a D₉₀-value of about 5.9 µm is complete. The particle size analyses were performed according to the test method as described in table 7.

Accordingly, in a preferred embodiment of the present invention the D₉₀-value of the particle size distribution of Candesartan cilexetil, being measured by laser diffraction, ranges from 1.0 µm to 10.0 µm. More preferred, the D₉₀-value of the particle size distribution of Candesartan cilexetil ranges from of 4.0 µm to 8.0 µm.

As already indicated above, it is economically not attractive to develop, register and manufacture all of the 16 theoretically possible fixed dose compositions of Amlodipine, Candesartan cilexetil and Hydrochlorothiazide.

It has been found that the set of fixed dose pharmaceutical compositions containing 5 mg/16 mg/12.5 mg, 10 mg/16 mg/12.5 mg, 5 mg/32 mg/25 mg, 10 mg/32 mg/25 mg Amlodipine/Candesartan cilexetil/Hydrochlorothiazide is advantageous from a regulatory and economically point of view.

The advantages are as follows:
• Only the amount of the active Amlodipine besilate is amended, whereas the amounts (in wt.-%) of the actives Candesartan cilexetil and Hydrochlorothiazide remain constant. As can be seen from table 4, this allows to produce all four different combinations of strengths from only one granular matrix for each composition, rendering the manufacturing process much more cost efficient
• If the total weight of the composition is 285 mg or more, the relative amount of Amlodipine besilate remains below 5 wt.-% compared to the total weight of the composition. If, in addition, Amlodipine besilate is solely replaced by a filler so that the total weight of the composition remains constant, there is no need to perform additional bioequivalence studies.

**Table 3**

| | **formulations Ic+IIc 5/16/12.5** | **formulations Ib+IIb 10/16/12.5** | **formulations Ia+IIa 5/32/25** | **formulations I+II 10/32/25** |
|---|---|---|---|---|
| **Total weight** | 285.00 mg | | 570.00 mg | |
| **Candesartan cilexetil** | 5.7% | | | |
| **Hydrochlorothiazide** | 4.4% | | | |
| **Amlodipine besilate** | 2.45% | 4.9% | 1.23% | 2.45% |
| **APIs in total** | 35.45 mg | 42.40 mg | 63.95 mg | 70.90mg |
| **Excipients in total** | 249.55 mg | 242.60 mg | 506.05 mg | 499.10 mg |
| **Restriction fulfilled** | quantitatively to formulation IV | Amlo: (i) + (iii) | Amlo: (i) + (iii) | Reference (supposed BE-strength) |

In an especially preferred embodiment of the present invention, HCT is only present in the granular matrix of the pharmaceutical composition. A further advantage of this embodiment is that the granular matrix comprising Candesartan cilexetil and HCT remains the same for all 4 combinations of strengths, which represents a major advantage for the manufacturing since only one kind of granules (instead of four different kinds of granules) has to be produced to satisfy all 4 combinations.

The invention also relates to a process for the manufacture of pharmaceutical compositions comprising Candesartan cilexetil, Hydrochlorothiazide and Amlodipine.

Tablets containing Candesartan cilexetil, Hydrochlorothiazide and Amlodipine besilate were prepared by compression of a blend comprising a granulate of Candesartan cilexetil, Hydrochlorothiazide and a first set of excipients with an admixed extragranular matrix comprising Amlodipine Besilate and a second set of excipients.

The dry components of the granulate, i.e. Candesartan cilexetil, polyethylene glycol, maize starch, lactose monohydrate, carboxymethyl cellulose (carmellose) calcium, hydroxypropyl cellulose (hyprolose, HPC) and Hydrochlorothiazide were sifted and mixed in a high shear rapid mixer and granulator (RMG) until a homogenous blend was obtained (Steps 1 to 3), then wetted with purified water and granulated (Step 4). The wet granulate was then subjected to a fluidized bed drying process to obtain a moisture level corresponding approximately to the starting moisture content of the dry ingredients mixture (Step 5).

The dry granulate was then milled through a Quadro^{®} Comil^{®} to obtain granules of uniform size (Step 6). The resulting granulate was mixed with the extragranular matrix components, i.e. Amlodipine besilate, microcrystalline cellulose and magnesium stearate (Steps 7 to 12), and then compressed using a rotary tablet machine (Step 13). The tablets were packed in opaque PVC/PVdC blisters and Alu-Alu blisters and charged on stability at 40ºC/75% RH for up to 3 months.

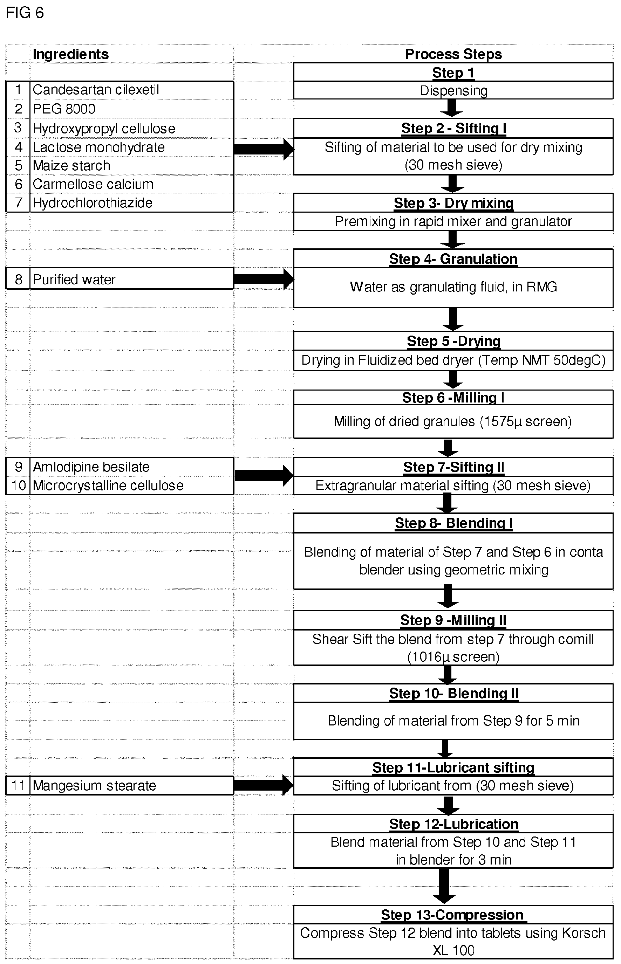

A detailed process for the manufacture of pharmaceutical compositions according to formulations I and la-c of the present invention is displayed in figure 6. The manufacture of pharmaceutical compositions according to formulations II and IIa-c of the present invention is practically identical with the exception that Hydrochlorothiazide is not added together with the other components of the granulate such as Candesartan cilexetil, but added together with the other extragranular matrix components such as Amlodipine besilate.

### EXAMPLES

### Pharmaceutical compositions

Tablets containing Amlodipine besilate, Candesartan cilexetil and Hydrochlorothiazide were prepared according to the process as shown in figure 1.

**Table 4**

| | | formulation Ic 5/16/12.5 | formulation Ib 10/16/12.5 | formulation Ia 5/32/25 | formulation I 10/32/25 |
|---|---|---|---|---|---|
| Ingredients | Function | Qty/Tab [mg] (wt.-%*) | | | |
| **Intragranular** | | | | | |
| Candesartan cilexetil | Active substance | 16.00 (5.61) | | 32.00 (5.61) | |
| PEG 8000 PF | Stabilizer | 2.60 (0.91) | | 5.20 (0.91) | |
| Lactose monohydrate Pharmatose 200M | Filler | 155.90 (54.70) | | 311.80 (54.70) | |
| Maize starch B | Filler | 28.00 (9.82) | | 56.00 (9.82) | |
| Carmellose calcium (ECG-505) | Disintegrant | 11.50 (4.04) | | 23.00 (4.04) | |
| Hydroxypropyl cellulose (Klucel) | Binder | 18.50 (6.49) | | 37.00 (6.49) | |
| Hydrochlorothiazide | Active substance | 12.50 (4.39) | | 25.00 (4.39) | |
| Total intragranular | - | 245 (85.96) | | 490 (85.96) | |
| **Extragranular** | | | | | |
| Amlodipine besilate | Active substance | 6.95 (2.45) | 13.90 (4.90) | 6.95 (1.23) | 13.90 (2.45) |
| Microcrystalline cellulose PH102 | Filler | 31.50 (11.05) | 24.55 (8.60) | 69.95 (12.27) | 63.00 (11.05) |
| Magnesium stearate | Lubricant | 1.55 (0.54) | | 3.10 (0.54) | |
| | | | | | |
| Total | - | 285.00 (100.00) | | 570.00 (100.00) | |

| | | | | | |
|---|---|---|---|---|---|
| * Relative amount, based on the weight of the total composition | | | | | |

As apparent from table 4, the set of up to four pharmaceutical compositions I, la, Ib and Ic may be manufactured by using the same granular matrix. In other words, there is no need to produce a different granular matrix for each composition of strengths, rendering the manufacturing process much more cost efficient. Only the amount of one active (i.e. Amlodipine besilate) in the extragranular matrix has to be adapted.

**Table 5**

| | | formulation IIc 5/16/12.5 | formulation IIb 10/16/12.5 | formulation IIa 5/32/25 | formulation II 10/32/25 |
|---|---|---|---|---|---|
| Ingredients | Function | Qty/Tab [mg] (wt.-%*) | | | |
| **Intragranular** | | | | | |
| Candesartan cilexetil | Active substance | 16.00 (5.61) | | 32.00 (5.61) | |
| PEG 8000 PF | Stabilizer | 2.60 (0.91) | | 5.20 (0.91) | |
| Lactose monohydrate Pharmatose 200M | Filler | 155.90 (54.70) | | 311.80 (54.70) | |
| Maize starch B | Filler | 28.00 (9.82) | | 56.00 (9.82) | |
| Carmellose calcium (ECG-505) | Disintegrant | 5.50 (1.93) | | 11.00 (1.93) | |
| Hydroxypropyl cellulose (Klucel) | Binder | 24.50 (8.60) | | 49.00 (8.60) | |
| Total Intragranular | - | 232.50 (83.33) | | 465.00 (83.33) | |
| **Extragranular** | | | | | |
| Hydrochlorothiazide | Active substance | 12.50 (4.39) | | 25.00 (4.39) | |
| Amlodipine besilate | Active substance | 6.95 (2.44) | 13.90 (4.88) | 6.95 (1.22) | 13.90 (2.44) |
| Microcrystalline cellulose PH102 | Filler | 26.50 (9.30) | 19.55 (6.86) | 69.95 (12.27) | 63.00 (11.05) |
| Magnesium stearate | Lubricant | 1.55 (0.54) | | 3.10 (0.54) | |
| | | | | | |
| Total | - | 285.00 (100.00) | | 570.00 (100.00) | |

| | | | | | |
|---|---|---|---|---|---|
| * Relative amount, based on the weight of the total composition | | | | | |

As apparent from table 5, the complete set of up to four pharmaceutical compositions II, IIa, IIb and IIc may be manufactured by using only one granular matrix. Again, there is no need to produce a different granular matrix for each composition of strengths, rendering the manufacturing process much more cost efficient. However, the amount of two actives (i.e. Amlodipine besilate and Hydrochlorothiazide) in the extragranular matrix has to be adapted.

### Dissolution testing

The in-vitro dissolution of tablets according was analyzed according to Ph. Eur. 2.9.3/USP <711> - Method 2 (Paddle Apparatus) with the settings as displayed in table 6.

**Table 6**

| **Drug Name** | **Reference** | **USP Apparatus** | **Speed [RPM[** | **Medium** | **Volume [ml]** | **Recommended Sampling Times [minutes]** |
|---|---|---|---|---|---|---|
| Amlodipine besylate | OGD and USP | II (Paddle) | 75 | 0.01 N HCl | 500 | 10,20,30,45 and 60 |
| Candesartan cilexetil | BP draft monograph | II (Paddle) | 50 | 0.03% w/v polysorbate 80 in buffer solution pH 7.2 | 900 | 45min (75%) |
| Candesartan cilexetil/HCTZ (16/12,5mg) | OGD | II (Paddle) | 50 | 0.35% polysorbate 20 in phosphate buffer pH 6.5 | 900 | 10,20,30,45 and 60 |
| Candesartan cilexetil (32mg); Candesartan Cilexetil/HCTZ (32/12,5mg and 32/25mg) | OGD | II (Paddle) | 50 | 0.70% polysorbate 20 in 0.05 M phosphate buffer pH 6.5 | 900 | 10,20,30,45 and 60 |
| Hydrochlorothiazide | USP | I (basket) | 100 | 0.1 N hydrochloric acid | 900 | 60 min (NLT 60%) |

### Particle size analysis

The particle size analyses were performed as described in table 7.

**Table 7**

| | | | |
|---|---|---|---|
| **Apparatus** | Model: | | Mastersizer 2000, Malvern |
| | Accessory: | | Scirocco 2000 (A) |
| **Parameters** | Particle RI: | | 1.63 |
| | Absorption: | | 0.1 |
| | Analysis model: | | General purpose |
| | Measurement time (Sample): | | 8 seconds |
| | Background: | | 8 seconds |
| | Sampling frequency: | | 50% |
| | Scattering pressure: | | 3.0 bar |
| | Obscuration: | | 0.5∼6% |
| **Test method** | 1) | Clean the sample plate Scirocco2000 (A) and dispersing funnel | |
| | 2) | Sample preparation: Make samples to be well mixed for 3 minutes (from top to bottom, left and right) | |
| | 3) | Take appropriate sample into injector plate and start CANDESARTAN CILEXETIL standard measurement procedure edited, repeat the testing 3 times. In each test intervals, vacuum purge 60 seconds with gas pressure to 3.0bar | |
| | 4) | After the testing, clean the sample plate and dispersing funnel with pressure to 2.0 bar and scattering sampling frequency to 50% | |
| | 5) | Measure the sample of each batch for three times | |

## Claims

1. A fixed dose pharmaceutical composition comprising the active substances Candesartan cilexetil, Amlodipine and Hydrochlorothiazide for the treatment of hypertension.

2. A fixed dose pharmaceutical composition according to claim 1 in the form of a tablet, comprising
• Candesartan cilexetil and a first matrix of excipients, and
• Amlodipine and a second matrix of excipients, comprising at least one filler,
wherein Hydrochlorothiazide may be comprised in either matrix.

3. A fixed dose pharmaceutical composition according to claim 2, wherein
a) the first matrix is in the form of granules comprising
• Candesartan cilexetil,
• one or more disintegrants,
• one or more binders,
• one or more fillers,
• one or more stabilizers,
• optionally one or more glidants, and
• optionally other pharmaceutically acceptable excipients;
b) the second, extragranular matrix comprises
• Amlodipine,
• one or more fillers,
• one or more lubricants, and
• optionally other pharmaceutically acceptable excipients;
wherein Hydrochlorothiazide may be present either in the first granular matrix or in the second, extragranular matrix.

4. A fixed dose pharmaceutical composition according any of the preceding claims, comprising one or more disintegrants being selected from carboxymethyl cellulose (carmellose), sodium starch glycolate, polyvinylpyrrolidone, crospovidone and croscarmellose, preferably carmellose calcium.

5. A fixed dose pharmaceutical composition according to any of the preceding claims, comprising one or more binders being selected from hydroxypropyl cellulose (HPC), hydroxypropyl methylcellulose (HPMC), dihydroxypropyl cellulose, methyl cellulose, hydroxyethyl cellulose, ethyl cellulose, sodium carboxymethyl cellulose, polyethylene glycol, maltodextrin, pregelatinized starch, polymethacrylates, sodium alginate, polyvinylpyrrolidone (povidone) and vinylpyrrolidone/vinylacetate copolymer (copovidone) and mixtures thereof, preferably hydroxypropyl cellulose (HPC).

6. A fixed dose pharmaceutical composition according any of the preceding claims, comprising one or more fillers being selected from lactose, starch, microcrystalline cellulose (MCC), sucrose, mannitol, dicalcium phosphate, calcium carbonate, magnesium carbonate, low substituted hydroxypropyl cellulose (L-HPC), powder cellulose, calcium silicate, calcium phosphate, sorbitol, dextrin, kaolin, magnesium oxide, calcium sulfate, xylitol, isomalt, glucose, fructose, maltose and mixtures thereof, preferably being selected from lactose monohydrate, maize starch, microcrystalline cellulose (MCC) and mixtures thereof.

7. A fixed dose pharmaceutical composition according any of the preceding claims, comprising one or more lubricants being selected from magnesium stearate, calcium stearate, sodium stearate, stearic acid, sodium glyceryl behenate, hexanedioic acid, hydrogenated vegetable oil sodium, stearyl fumarate, glycerin fumarate and mixtures thereof, preferably magnesium stearate.

8. A fixed dose pharmaceutical composition according to any of claims 3 to 7, wherein
• the amount of disintegrant(s) ranges from 1.5 wt.-% to 4.0 wt.-%,
• the amount of binder(s) ranges from 1.0 wt.-% to 10.0 wt.-%,
• the ratio between the amount of disintegrant(s) and the amount of binder(s) is in the range of from 1.0:1.5 to 1.0:4.0 by weight;
all wt.-%values being relative to the total weight of the composition.

9. A fixed dose pharmaceutical composition according to any of claims 3 to 8, wherein
• the disintegrant is carmellose calcium,
• the binder is hydroxypropyl cellulose (HPC),
• the fillers in the granules are lactose and starch,
• the stabilizer is polyethylene glycol (PEG),
• the extragranular filler is microcrystalline cellulose (MCC), and
• the lubricant is magnesium stearate.

10. A fixed dose pharmaceutical composition according to any one of the preceding claims, comprising polyethylene glycol (PEG) in a ratio of from 1.0:6.0 to 1.0:8.0 by weight relative to Candesartan cilexetil.

11. A fixed dose pharmaceutical composition according to any one of the preceding claims, wherein the D₉₀-value of the particle size distribution of Candesartan cilexetil ranges from 1.0 µm to 10.0 µm, preferably from of 4.0 µm to 8.0 µm.

12. A fixed dose pharmaceutical composition according to any one of the preceding claims, comprising
• 5 mg or 10 mg Amlodipine,
• 4 mg, 8 mg, 16 mg or 32 mg Candesartan cilexetil, and
• 12.5 mg or 25 mg Hydrochlorothiazide.

13. A set of fixed dose pharmaceutical compositions according to claim 12, comprising 5 mg/16 mg/12.5 mg, 10 mg/16 mg/12.5 mg, 5 mg/32 mg/25 mg and 10 mg/32 mg/25 mg Amlodipine/Candesartan cilexetil/Hydrochlorothiazide.

14. A set of fixed dose pharmaceutical composition according to claim 12 or claim 13, wherein
• the relative amounts of Candesartan cilexetil and Hydrochlorothiazide in wt.-% compared to the total weight of the composition remain constant in each composition,
• Amlodipine besilate is solely replaced by a filler, so that the total weight of the composition remains constant, and
• the total weight of the composition is 285 mg or more, so that the relative amount of Amlodipine besilate remains below 5 wt.-% compared to the total weight of the composition.

15. A process for the manufacture of a pharmaceutical composition according to one of the preceding claims, comprising the steps of:
(a) Preparing a homogenous blend comprising Candesartan cilexetil, Hydrochlorothiazide and a first set of excipients, comprising one or more disintegrant(s), one or more binder(s), one or more filler(s) and one or more stabilizer(s);
(b) Preparing granules by means of wet granulation;
(c) Drying the wet granules and sieving;
(d) Blending the granules with Amlodipine besilate and a second set of excipients, comprising one or more filler(s) and one or more lubricant(s);
(e) Compressing the final blend into tablets.
